# EUROPEAN PATENT SPECIFICATION

(11) **EP 1 804 848 B1**
(45) Date of publication and mention of the grant of the patent: **15.07.2015**
(21) Application number: 05815562.3
(22) Date of filing: 19.10.2005
(51) Int. Cl.: A61L 31/10, A61L 31/16

(54) **IMPLANTABLE DEVICES COMPRISING BIOLOGICALLY ABSORBABLE STAR POLYMERS AND METHODS FOR FABRICATING THE SAME**
IMPLANTIERBARE VORRICHTUNGEN MIT BIOLOGISCH ABSORBIERBAREN, STERNFÖRMIGEN POLYMEREN UND VERFAHREN ZU IHRER HERSTELLUNG
DISPOSITIFS IMPLANTABLES COMPRENANT DES POLYMERES EN ETOILE BIOLOGIQUEMENT ABSORBABLES, PROCEDES DE FABRICATION DE CES DERNIERS

(30) Priority: 29.10.2004 US 978031
(43) Date of publication of application: 11.07.2007
(73) Proprietor: Abbott Cardiovascular Systems Inc., Santa Clara, CA 95054 (US)
(72) Inventor: PACETTI, Stephen, D., San Jose, CA 95130 (US)
(74) Representative: Boult Wade Tennant
(86) International application number: PCT/US2005/037987
(87) International publication number: WO 2006/049907

(56) References cited:
- WO-A-98/47948
- WO-A-03/061631
- DE-A1- 19 849 464
- US-B1- 6 338 904

## Description

### BACKGROUND

### 1. Field of the Invention

This invention is directed to coatings for drug delivery devices, such as drug-eluting vascular stents, and methods for producing the same.

### 2. Description of the State of the Art

Percutaneous transluminal coronary angioplasty (PTCA) is a procedure for treating heart disease. A catheter assembly having a balloon portion is introduced percutaneously into the cardiovascular system of a patient via the brachial or femoral artery. The catheter assembly is advanced through the coronary vasculature until the balloon portion is positioned across the occlusive lesion. Once in position across the lesion, the balloon is inflated to a predetermined size to radially compress against the atherosclerotic plaque of the lesion to remodel the lumen wall. The balloon is then deflated to a smaller profile to allow the catheter to be withdrawn from the patient's vasculature.

A problem associated with the above procedure includes formation of intimal flaps or torn arterial linings, which can collapse and occlude the conduit after the balloon is deflated. Moreover, thrombosis and restenosis of the artery may develop over several months after the procedure, which may require another angioplasty procedure or a surgical by-pass operation. To reduce the partial or total occlusion of the artery by the collapse of arterial lining, and to reduce the chance of the development of thrombosis and restenosis, a stent is implanted in the lumen to maintain the vascular patency.

Stents are used not only as a mechanical intervention but also as a vehicle for providing biological therapy. As a mechanical intervention, stents act as scaffoldings, functioning to physically hold open and, if desired, to expand the wall of the passageway. Typically, stents are capable of being compressed, so that they can be inserted through small vessels via catheters, and then expanded to a larger diameter once they are at the desired location. Examples in patent literature disclosing stents which have been applied in PTCA procedures include stents illustrated in U.S. Patent No. 4,733,665 issued to Palmaz, U.S. Patent No. 4,800,882 issued to Gianturco, and U.S. Patent No. 4,886,062 issued to Wiktor.

Biological therapy can be achieved by medicating the stents. Medicated stents provide for the local administration of a therapeutic substance at the diseased site. In order to provide an efficacious concentration to the treated site, systemic administration of such medication often produces adverse or toxic side effects for the patient. Local delivery is a preferred method of treatment in that smaller total levels of medication are administered in comparison to systemic dosages, but are concentrated at a specific site. Local delivery thus produces fewer side effects and achieves more favorable results. One proposed method for medicating stents involves the use of a polymeric carrier coated onto the surface of a stent. A solution which includes a solvent, a polymer dissolved in the solvent, and a therapeutic substance dispersed in the blend is applied to the stent. The solvent is allowed to evaporate, leaving on the stent surface a coating of the polymer and the therapeutic substance impregnated in the polymer.

Local administration of therapeutic agents via stents has shown favorable results in reducing restenosis. However, to the extent that the functionality of stents has been optimized in recent years, stents still can cause some undesirable effects. For example, the continued exposure of a stent to blood can lead to thrombus formation, and the presence of a stent in a blood vessel can, over time, cause the blood vessel wall to weaken, which creates the potential for an arterial rupture or the formation of aneurisms. A stent can also become so overgrown by tissue after its implantation that its usefulness may be substantially decreased while its continued presence may cause a variety of problems or complications. It is therefore desirable for the stent to be biodegradable or bioabsorbable so as to diminish the adverse risks that would otherwise be associated with the stent's continued presence once its usefulness at the treatment site has ceased or has at least become substantially diminished.

To obviate such complications, stents can be fabricated of materials that are biodegradable or bioabsorbable. It is necessary to select a material that is both biodegradable and biocompatible, and has suitable physical and mechanical properties such as sufficient strength, the radial flexibility necessary for the stent to undergo expansion, and longitudinal flexibility to allow the stent to be advanced through a contorted vasculature and to adapt to a non-linear deployment site. In addition, it is desirable to have the biologically absorbable stent coatings having biologically beneficial properties to also have a tunable absorption rate, a drug release rate that can be modulated, and good mechanical properties.

In addition, using traditional polymers for fabricating stent coatings may create some other difficulties. For example, the viscosity of a polymer solution commonly used for depositing coatings on stents can be quite high even when the concentration of the polymer in the solution is relatively low. This is because in order to have good mechanical properties such as ultimate elongation, the polymer molecular weight must be above a certain value, Mₙ = 50 K Daltons for example. Moreover, much of the manufacturing time in coating drug-eluting stents, can consist of applying multiple applications of a coating, with drying in between, in order to build up the desired thickness. The coating cannot be applied all at once due to issues with coating defects and webbing in between the struts. A coating, which could be applied at higher percent solids, would shorten the coating time. In some embodiments of drug-eluting stents, the drug/polymer reservoir is placed into some type of depot on the stent. Filling these depots can require multiple filling and drying cycles as most polymer coating formulations are only a few percent (<10%) solids. It is, therefore, desirable to have polymers that are both suitable for making stent coatings and are capable of forming solutions having the same or higher polymer concentration, but having reduced viscosity compared to traditional polymers.

WO 98/47948 discloses compositions for coating biological and non-biological surfaces, which minimise or prevent cell-cell contact and tissue adhesion, and methods of preparation and use thereof.

US 6,338,904 discloses bio-active coating compositions for polymeric substrates.

WO 03/061631 discloses a highly lubricious hydrophilic coating for a medical device comprising a mixture of colloidal aliphatic polyurethane, an aqueous dilution of PVP and specific dendrimers to enhance the physical integrity of the coating, to improve adhesion and to covalently bind or load one of a certain antithrombolitic drug or a certain antibiotic drug or other agent within the dendrimous structure.

DE 198 49 464 discloses a stent coated with a carrier polymer, to which cyclodextrin is attached.

US 6,818,247 discloses a composition comprising an ethylene vinyl alcohol copolymer and a dimethyl acetamide solvent for forming a coating on prostheses such as stents.

### SUMMARY

The invention of this document is directed to an article comprising:
an implantable substrate; and
a coating deposited on at least a portion of the substrate, wherein the coating comprises:
   a star polymer;
   a comb polymer wherein the sidechains are at least 10% of the contour length of the polymer backbone; or
   a brush polymer wherein the sidechains are at least 10% of the contour length of the polymer backbone,
wherein the star polymer, the comb polymer or the brush polymer each comprise any one or any combination of biologically absorbable polymers and durable polymers,
wherein the star polymer comprises any one or any combination of poly(D,L-lactic acid), poly(D-lactic acid), poly(L-lactic acid), poly(L-lactide-co-D,L-lactide), poly(glycolide), poly(D,L-lactide-co-glycolide), poly(caprolactone), poly(D,L-lactide-co-caprolactone), poly(L-lactide-co-caprolactone), poly(glycolide-co-caprolactone), poly(3-hydroxybutyrate), poly(4-hydroxybutyrate), poly(3-hydroxyvalerate), poly(hydroxybutyrate-co-valerate), poly(dioxanone), poly(trimethylene carbonate), poly(D,L-lactide-co-trimethylene carbonate), n-star poly(n-butyl methacrylate) and n-star poly(isobutylene-co-block-styrene), and
wherein the comb polymer or the brush polymer each comprise any one or any combination of poly(D,L-lactic acid), poly(D-lactic acid), poly(L-lactic acid), poly(L-lactide-co-D,L-lactide), poly(glycolide), poly(D,L-lactide-co-glycolide), poly(caprolactone), poly(D,L-lactide-co-caprolactone), poly(L-lactide-co-caprolactone), poly(glycolide-co-caprolactone), poly(3-hydroxybutyrate), poly(4-hydroxybutyrate), poly(3-hydroxyvalerate), poly(hydroxybutyrate-co-valerate), poly(dioxanone), poly(trimethylene carbonate), and poly(D,L-lactide-co-trimethylene carbonate).

In some embodiments the substrate is a stent, such as an intravascular stent. The polymers can be durable or biologically absorbable and can comprise any one or any combination of poly(D,L-lactic acid), poly(D-lactic acid), poly(L-lactic acid), poly(L-lactide-co-D,L-lactide), poly(glycolide), poly(D,L-lactide-co-glycolide), poly(caprolactone), poly(D,L-lactide-co-caprolactone), poly(L-lactide-co-caprolactone), poly(glycolide-co-caprolactone), poly(3-hydroxybutyrate), poly(4-hydroxybutyrate), poly(3-hydroxyvalerate), poly(hydroxybutyrate-co-valerate), poly(dioxanone), poly(trimethylene carbonate), and poly(D,L-lactide-co-trimethylene carbonate). Some embodiments select the polymer to specifically exclude any one or any combination of the polymers listed here.

In some embodiments the polymer comprises homopolymer, copolymers, or terpolymers. Some embodiments select the polymer to specifically exclude any one or any combination of the polymers listed here. Some of these are selected from poly(n-butyl methacrylate) and poly(isobutylene-co-block-styrene). Some embodiments select the polymer to specifically exclude any one or any combination of the polymers listed here.

The polymers can be prepared by
ring-opening (co)polymerization,
radical (co)polymerization,
ionic (co)polymerization,
living cationic (co)polymerization;
anionic polymerization; or
living polymerization techniques (including atom transfer (co)polymerization, metallocene catalysis, Ziegler-Natta catalysis, nitroxide mediated techniques, or iniferter techniques),
wherein the polymerization is of one or more monomer types.

For articles comprising brush and comb polymers, some embodiments comprise 1 to 1000 or 5 to 500 sidechains.

Methods of making these articles and methods of using these articles are also encompassed by this disclosure

### DETAILED DESCRIPTION

### 1. Terms and Definitions.

The term "polymer" is defined to be inclusive of homopolymers and copolymers (further defined below), including any of the following:
random copolymers;
alternating copolymers;
cross-linked polymers;
linear polymers;
thermoplastic polymers; and
block-copolymers.

The term "polymer" is further defined as a synonym of the term "polymeric compound." The term "copolymer" is defined as a polymer derived from more than one species of monomer, including copolymers that are obtained by copolymerization of two monomer species, those obtained from three monomers species ("terpolymers"), etc.

The term "random copolymer" is defined as a copolymer containing macromolecules in which the probability of finding a given monomeric unit at any given site in the chain is substantially independent of the nature of the adjacent units.

The term "alternating copolymer" is defined as a copolymer containing macromolecules comprising two species of monomeric units in alternating sequence.

The term "cross-linked polymer" is defined as a polymer that includes cross-links. "Cross-link" is defined as a region in a macromolecule from which at least four chains emanate, and which is formed by reactions involving sites or groups on existing macromolecules or by interactions between existing macromolecules. The region defining the "cross-link" can be an atom, a group of atoms, or a number of branch points connected by bonds, groups of atoms, or oligomeric chains.

The term "linear polymer" is defined as a macromolecule, the structure of which comprises a multiple repetition, in linear sequence, of units derived from low relative molecular mass molecules.

The term "thermoplastic polymer" is defined as a polymer that does not readily form cross-links, even when heated, whether in the presence of a cross-linking catalyst or in the absence of such catalyst. Typically, the cross-links are not formed because the thermoplastic polymers substantially lack reactive chemical fragments in their macromolecules needed for forming the cross-link. As a result, a thermoplastic polymer can be softened and fused when heated and hardened when cooled. Cycles of melting and solidifying a thermoplastic polymer can be repeated many times without the polymer undergoing any appreciable chemical change. Also, a thermoplastic polymer that is soluble in a solvent remains soluble in that solvent after being subjected to many melting-solidifying cycles.

Polymers that are both linear and thermoplastic are defined as "linear thermoplastic polymers." For the purposes of some embodiments of the present invention, whenever the terms "linear polymer," or "thermoplastic polymer," or "linear thermoplastic polymer" are used, these terms specifically exclude materials with cross-links. In other words, as used in some embodiments of the present invention, "linear polymers" and "thermoplastic polymers" are substantially free of cross-linked fragments.

The term "block copolymer" is defined as a copolymer containing a linear arrangement of blocks, a block being defined as a portion of a polymer molecule in which the monomeric units have at least one constitutional or configurational feature absent from adjacent portions. For example, a block copolymer of moiety A and moiety B may be written as -A-A-A-B-B-B-B-. Such block copolymer is often referred to as an "AB block copolymer." The blocks need not be linked on the ends, since the individual blocks are usually long enough to be considered polymers in their own right. The AB block copolymer can be, accordingly, named poly(A-block-B-block). The term "block copolymer" is intended to broadly include copolymers having two or more types of blocks, for example, di-block and tri-block copolymers.

The terms "biologically absorbable, bioabsorbable, biodegradable, bioresorbable, bioerodable, absorbable, degradable, resorbable, or erodable coatings and/or polymers is defined as coatings or polymers that are capable of being substantially to completely degraded, dissolved, or eroded when exposed to bodily fluids such as blood and that are gradually resorbed, absorbed or eliminated by the body. The processes of degradation, dissolution, erosion, and eventual absorption and elimination of the coating or polymer can be caused, for example, by hydrolysis, enzymatic action, oxidation, phagocytosis, metabolic processes, bulk or surface erosion, and the like.

Whenever reference is made to "biologically absorbable, bioabsorbable, biodegradable, bioresorbable, bioerodable, absorbable, degradable, resorbable, or erodable " stent coatings or polymers forming such stent coatings, it is understood that after the process of degradation, dissolution, erosion, absorption, or resorption has been substantially completed, substantially no coating will remain on the stent.

The term "durable polymers" is defined as polymers that are intended to remain in the body permanently or in some embodiments greater than 1, 2, or 3 years. They have limited mechanisms by which the polymer backbone may be cleaved in vivo by hydrolytic, enzymatic, or oxidative means. They are not "biologically absorbable" as defined above.

The term "star polymer" is defined as a polymer composed of star macromolecules, in accordance with the IUPAC Definition 2.32. "Star macromolecules" are defined as macromolecule containing relatively few branch points from which substantially linear chains or arms emanate.

For the purposes of the present invention, "star macromolecules" include "n-star macromolecules," "regular star macromolecules," and "variegated star macromolecules." An "*n-*star macromolecule" contains n, substantially linear chains or arms attached to the branch point, e.g., a three-star macromolecule, if n = 3. A "regular star macromolecule" contains substantially identical arms. A "variegated star macromolecule" contains substantially different arms such as difference in the composition of monomeric units composing the arms, e.g., the arms may be different in respect to their constitution or degree of polymerization.

The advantage to using star polymers is that they have a lower solution viscosity (for a given percent solids) or a lower melt viscosity. Moreover, they retain good mechanical properties due to their entanglement. These features are also present in comb and brush polymers when the brushes or combs extending from the polymer backbone have chain lengths similar to that of the backbone itself. In some embodiments, the contour length of the side brush or comb is at least 10, 20, 30, 40, 60, 80, or 90% of the contour length of the main polymer backbone.

These types of comb and brush polymers can also be used instead of star polymers if the brushes or combs extending from the polymer backbone are at least 10% of the contour length of the main polymer backbone itself.

The terms "ring-opening polymerization or copolymerization," also used as "ring-opening (co)polymerization" are defined as:
with respect to the ring-opening polymerization, a process of polymerization in which a cyclic monomer yields a monomeric unit which is acyclic or contains fewer rings than the monomer;
with respect to the ring-opening copolymerization, a process of copolymerization of more than one monomer that is a ring-opening polymerization with respect to at least one monomer.

The term "radical polymerization or copolymerization," also used as "radical (co)polymerization" is defined as a chain polymerization or copolymerization, respectively, in which the kinetic-chain carriers are radicals. "Chain polymerization or copolymerization" is a chain reaction in which the growth of a polymer chain proceeds substantially exclusively by reaction(s) between monomer(s) and reactive site(s) on the polymer chain with regeneration of the reactive site(s) at the end of each step.

The term "ionic polymerization or copolymerization," also used as "ionic (co)polymerization" is defined as a chain polymerization or copolymerization, respectively in which substantially all of the kinetic-chain carriers are ions or ion-pairs. "Anionic polymerization or copolymerization" (or "anionic (co)polymerization") and "cationic polymerization or copoly-merization" (or "cationic (co)polymerization)" are particular cases of "ionic (co)polymerization" where the ions or ion-pairs are anions and cations, respectively.

The term "living polymerization or copolymerization" (or "living (co)polymerization") is defined as a chain polymerization or copolymerization from which chain transfer and chain termination are substantially absent.

The term "polycondensation or copolycondensation," also used as "(co)polycondensation" is defined as a polymerization or copolymerization in which the growth of polymer chains substantially proceeds by condensation reactions between molecules of all degrees of polymerization.

### 2. Embodiments of the Invention.

According to embodiments of the invention, a star polymer can be deposited on at least a portion of a stent to form a stent coating. One suitable example of the general structure is schematically illustrated by Structure I. In Structure I, X is a central moiety serving as a focal branching point from which polymeric chains or arms emanate.

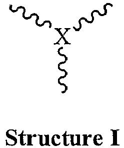

Useful polymeric arms can comprise linear polymers of various compositions, including linear homopolymers, linear copolymers or linear terpolymers. In some embodiments, useful polymeric arms include polymers formed as a result of any of the following polymerization, processes using one or more monomer(s):
ring-opening polymerization or copolymerization;
radical polymerization or copolymerization, e.g., using a trifunctional free radical initiator;
ionic polymerization or copolymerization, including cationic polymerization or copolymerization and anionic polymerization or copolymerization; and
living polymerization.

The stent coating can be a multi-layer structure that can include any of the following four layers or combination thereof:
a primer layer;
a drug-polymer layer (also referred to as "reservoir" or "reservoir layer");
a polymer-free drug layer; and
a topcoat layer.

Each stent coating layer can be formed by dissolving a star polymer or star-polymer blend in a solvent, or a solvent mixture, and applying the solution to the stent by spraying the stent or immersing it in the solution. After application, the solvent evaporates, drying the material. Drying can be accelerated by conducting at an elevated temperature or reduced pressure or both.

To incorporate a drug into the reservoir layer, the drug can be combined with the polymer solution described above. Alternatively, a polymer-free reservoir can be made. To fabricate a polymer free reservoir, the drug can be dissolved in a suitable solvent or solvent mixture and the resulting solution can be applied to the stent.

Instead of introducing the drug as a solution, the drug can be introduced as a colloidal system, such as a suspension or dispersion in an appropriate solvent. To make the suspension, the drug can be dispersed in the solvent phase using conventional colloidal chemistry techniques. Depending on a variety of factors, e.g., the nature of the drug, those having ordinary skill in the art can select the appropriate solvent phase of the suspension, as well as the quantity of drug to be dispersed in the solvent phase. The suspension can be mixed with a polymer solution, and the mixture can be applied to the stent as described above. Alternatively, the drug suspension can be applied on the stent without being mixed with the polymer solution.

The drug-polymer layer can be applied directly onto at least a part of the stent surface to serve as a reservoir for at least one active agent or a drug, which is incorporated into the reservoir layer. The optional primer layer can be applied between the stent and the reservoir to improve the adhesion of the drug-polymer layer to the stent. The topcoat layer, if used, can be applied over at least a portion of the reservoir and can serve as a rate-limiting membrane, which helps to control the drug's release. In one embodiment, the topcoat layer can be essentially free from any active agents or drugs. In another embodiment, the topcoat layer contains active agents in order to alter the drug-release profile or the drug permeability of the topcoat.

In one embodiment, any or all of the layers of the stent coating can be made of a star polymer. In another embodiment, the outermost layer of the coating can be limited to such a. polymer. To illustrate in more detail, in the stent coating having all three layers described above (i.e., the primer, the reservoir, and the topcoat layer), the outermost layer of the stent coating is the topcoat layer, which can comprise a star polymer. In this case, optionally, the remaining layers (i.e., the primer and the reservoir) can also comprise a star polymer; and each layer can comprise the same star polymer as on a different star polymer from the other layers. The stent coating can comprise any combination of primer layers, drug layers and topcoat layers and each of these can contain similar or dissimilar polymers.

According to embodiments of the present invention, star polymers belonging to one of two classes can be used to fabricate any or all layers of a stent coating: (1) biologically absorbable star polymers; and/or (2) durable star polymers. These two classes of star polymers can be described as follows.

### 1. Biologically Absorbable Star Polymers

To make biologically absorbable star polymers, special synthetic techniques can be used. Generally, useful biologically absorbable star polymers may have between 3 and 24 arms in the case where there is a central moiety "X," Alternatively, the polymers may have 5-20, or 10-15 arms. Particularly, the biologically absorbable star polymers can be obtained by ring-opening (co)polymerization, by free radical (co)polymerization, or by living (co)polymerization of a suitable monomer or monomers. Any monomer(s) capable of forming a biologically absorbable star (co)polymer via ring-opening polymerization or polycondensation can be used.

Examples of biologically absorbable star polymers that can be made by ring-opening polymerization or polycondensation and used include poly(D,L-lactide), poly(D-lactide), poly(L-lactide), poly(L-lactide-co-D,L-lactide), poly(glycolide), poly(D,L-lactide-co-glycolide), poly(caprolactone), poly(L-lactide-co-caprolactone), poly(glycolide-co-caprolactone), poly(3-hydroxypropionate), poly(3-hydroxy-2-methylpropionate), poly(3-hydroxybutyrate), poly(4-hydroxybutyrate), poly(3-hydroxyvalerate), poly(hydroxybutyrate-co-valerate), poly(dioxanone), poly(trimethylene carbonate), poly(D,L-lactide-co-trimethylene carbonate). Following the process of ring opening polymerization, the resulting star polymer can be applied on a stent to form a stent coating as described above.

Each of these polymers can be synthesized in a star form using the technique of ring-opening (co)polymerization or (co)polycondensation in the presence of a multifunctional initiator. The process of ring-opening polymerization can be illustrated for making n-star poly(D,L-lactides) as follows. The process of synthesis of other biologically absorbable star polymers listed above can be similar.

The synthesis of *n*-star poly(D,L-lactides) includes ring-opening polymerization of lactide (a cyclic dimer of lactic acid), in the presence of an *m*-functional initiator leading to formation of a variety of *n*-star poly(D,L-lactides), both regular and variegated. The term "*m-*functional initiator" is defined as an initiator having *m* reactive functional groups, where *m* is an integer having the value of 3 or higher. An example of reactive functional groups in the initiator is hydroxyl.

The number *n* of arms in the final *n*-star poly(D,L-lactide) can correspond to the value of *m*. To illustrate, to make a 3-armed poly(D,L-lactide) (*n* = 3), a trifunctional initiator (*m* = 3) can be used. To make a 4-armed poly(D,L-lactide) (*n* = 4), a tetrafunctional initiator (*m* = 4) can be used, and so on.

One example of a particular *n*-star poly(D,L-lactide) that can be synthesized and used is a 3-armed poly(D,L-lactide), which is a three-star polymer shown below as Polymer I:

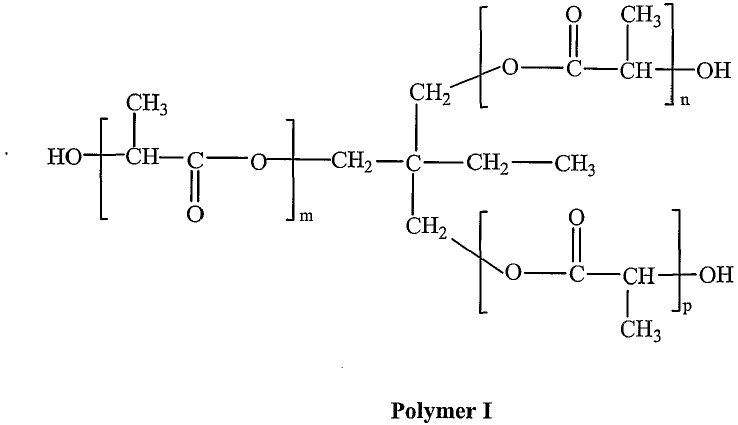

In Polymer I, the length of the three arms is determined by the value of integers "m," "n," and "p," which can be the same or different.

A 3-armed poly(D,L-lactide) (A) can be obtained by ring-opening polymerization of lactide, in the presence of a trifunctional initiator such as 1,1,1-trimethylolpropane or glycerol. The process, which can be catalyzed by stannous octoate, can be summarized schematically as Reaction I:

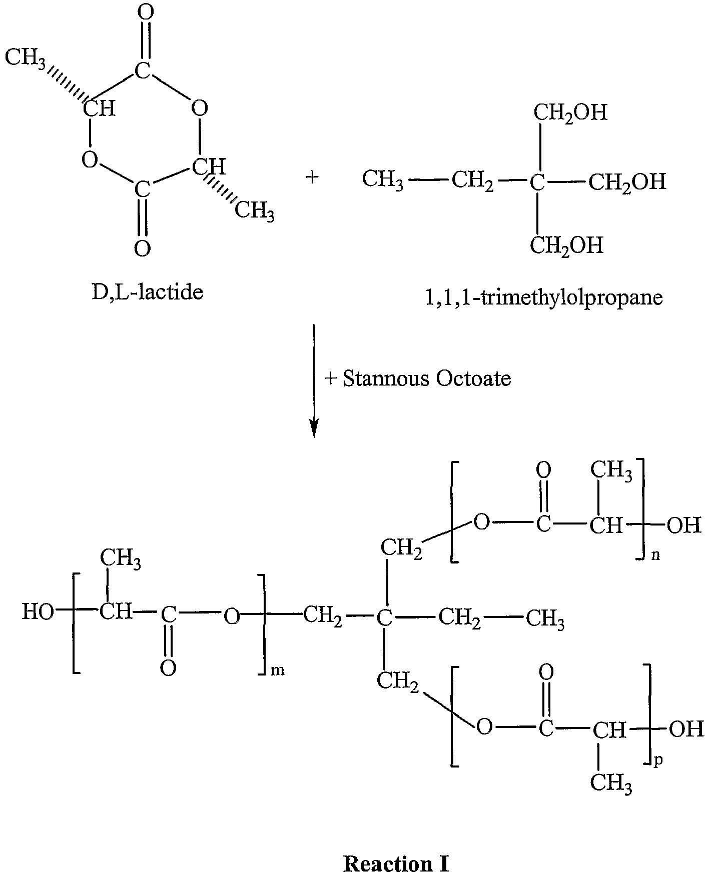

The polymerization may be carried out neat or in a solvent such as toluene. Typical conditions for the ring opening polymerization is a temperature of 60°C to 140°C for 1 to 24 hours. A useful catalyst is stannous octoate, but those of ordinary skill in the art can supply others. Useful catalyst to initiator ratios are 0.1/1 to 2/1 on a molar basis.

Another example of a particular *n*-star poly(D,L-lactide) that can be synthesized and used is a 4-armed poly(D,L-lactide), which is a four-star polymer (Polymer II):

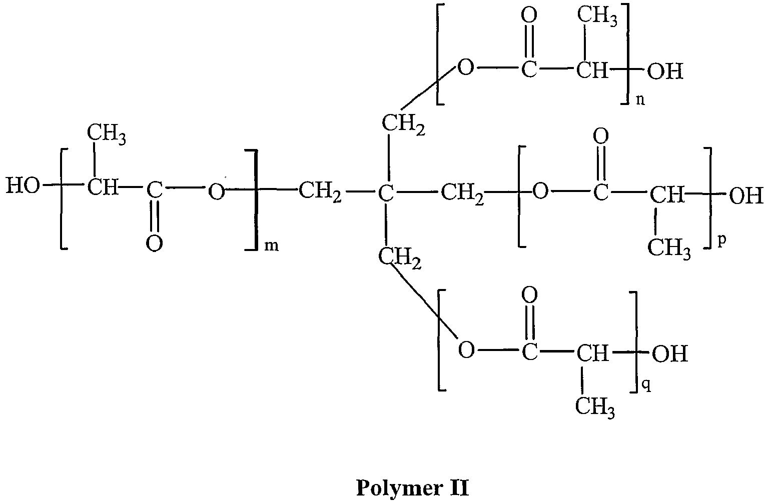

In Polymer II, the length of the four arms is determined by the value of integers "m," "n," "p," and "q," which can be the same or different.

A 4-armed poly(D,L-lactide) Polymer II can be obtained by ring-opening polymerization of lactide. The process is similar to the process shown by Reaction I, except that instead of a trifunctional initiator used in Reaction I, the process can be carried in the presence of a tetrafunctional initiator, for example, pentaerythritol (tetramethylolmethane). The process can be also catalyzed by stannous octoate and can be summarized schematically as Reaction II:

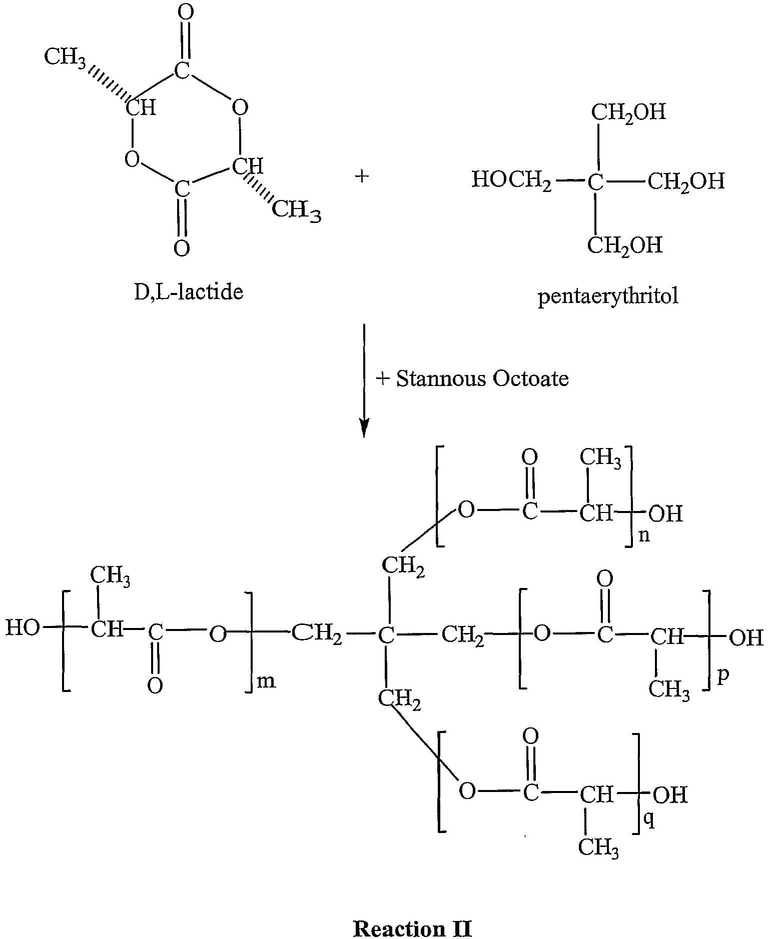

Star poly(D,L-lactide) having larger number of arms can be made in a manner that is similar to the procedure described above. For example, a 6-armed poly(D,L-lactide) can be made using a hexafunctional initiator, such as an α-cyclodextrin. Other hexafunctional, or higher, polyhydric initiators may be low molecular weight poly(3-hydroxymethyl propionate), or dendrimers of 2,2-bis-hydroxymethyl-propionic acid.

### 2. Durable Star Polymers

Generally, the durable star polymers that can be made and used can have between 3 and 24 arms. The durable star polymers can be synthesized using various techniques, for example, ionic (co)polymerization, living polymerization, or free radical polymerization. For example, the durable star polymers can be synthesized by ionic (co)polymerization, such as living cationic (co)polymerization or anionic polymerization, or by living polymerization techniques including atom transfer (co)polymerization, metallocene catalysis, Ziegler-Natta catalysis, nitroxide mediated, or iniferter techniques on any monomer or monomers capable of being (co)polymerized by these techniques.

Typically, the process of (co)polymerization leading to the formation of a durable star polymer can be carried out in the presence of an *m*-functional initiator defined above. The number *n* of arms in the final *n*-star durable polymer can correspond to the value of *m.* To illustrate, to make a 3-armed *n*-star durable polymer (*n* = 3), a trifunctional initiator (*m* = 3) can be used. To make a 4-armed *n*-star durable polymer (*n* = 4), a tetrafunctional initiator (*m* = 4) can be used, and so on. Those having ordinary skill in the art can select the monomer(s) that can be polymerized to form durable star polymers and conditions under which the synthesis can be carried out. Typical conditions to be chosen include the kind and the quantity of an appropriate initiator, catalyst, temperature, reaction time, and so on.

One example of a particular n-star durable polymer that can be synthesized and used is *n*-star poly(*n*-butyl methacrylate) (PBMA), such as 3-armed PBMA, which is a three-star polymer shown below as Polymer III:

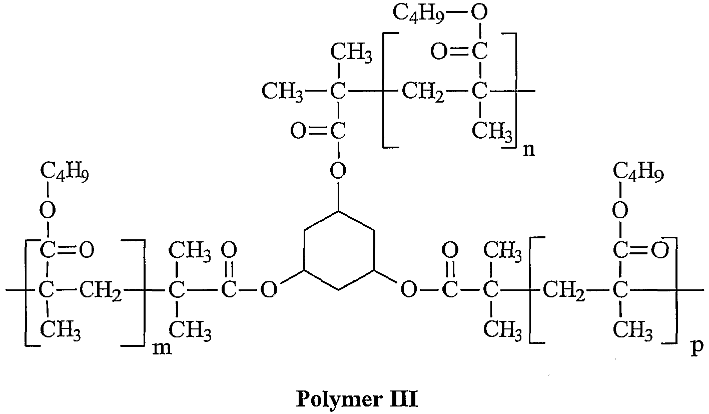

A 3-armed PBMA, Polymer III, can be obtained by the process of atom transfer polymerization of n-butyl methacrylate monomer, in the presence of a trifunctional initiator such as 1,3,5-*tris*-(cyclohexanoyl-2-bromo-isobutyrate). This initiator can be separately synthesized first, by reacting three equivalents of 2-bromoisobutyryl bromide with 1,3,5-cyclohexanetriol in the presence of a suitable base such as triethyamine to absorb the liberated HBr. The polymerization can be catalyzed by a copper bromide (I)/2,2'-bipyridine complex, can be summarized schematically as Reaction III:

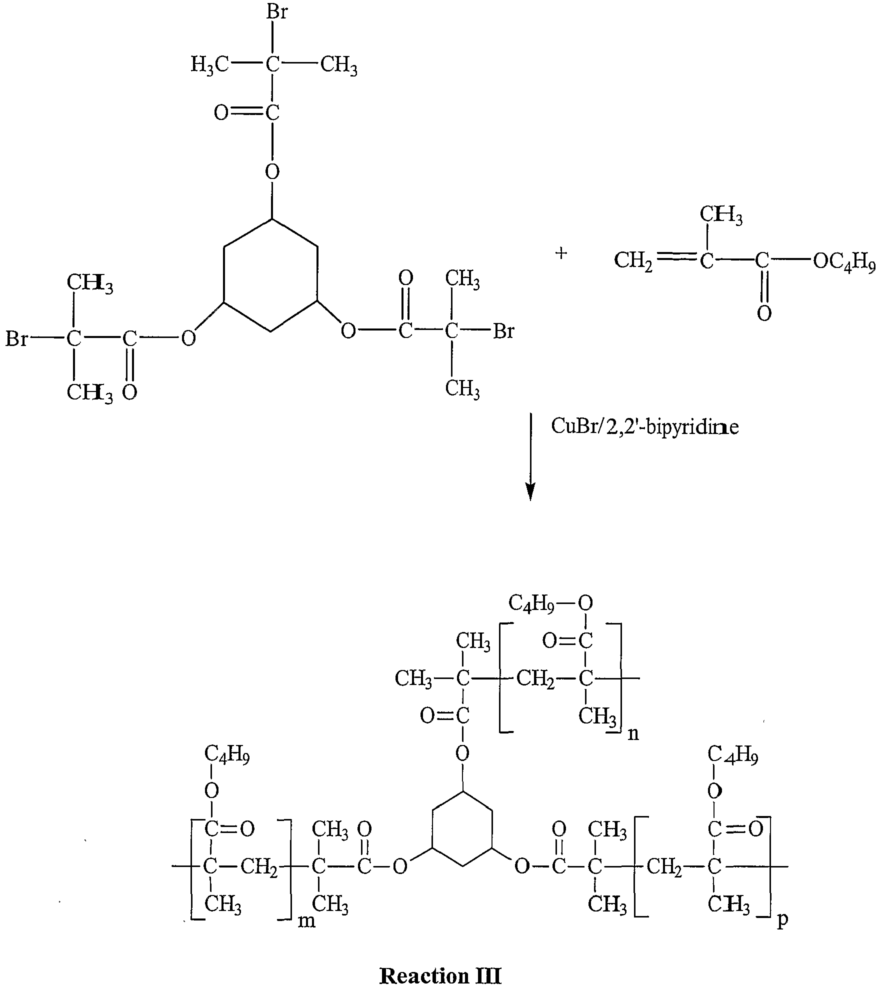

Appropriate conditions are ambient temperature to 110°C for 0.5 to 4 hours under an inert atmosphere such as argon. The reaction may be done neat or in solvents such as benzene, 2-butanone, chlorobenzene, or diphenylether. Useful monomer to initiator ratios are 10/1 to 1000/1. Additional synthetic details may be found in V. Coessens et al. Prog. Polym. Sci. 26 (2001) 337-377.

Other examples of durable *n*-star polymers that can be synthesized and used include *n*-star polymers in which linear branch arms comprise copolymers, for example block-copolymers, such as star poly(isobutylene-co-block-styrene) (PIBS), which is a three-star block-copolymer shown below as Polymer IV:

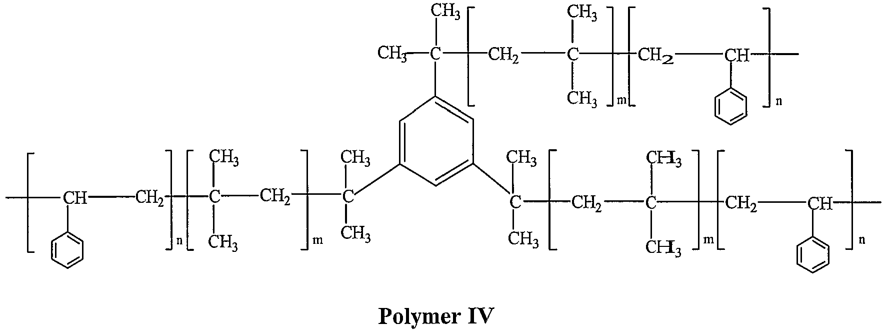

A 3-armed PIBS, Polymer IV, can be obtained by the process of living cationic copolymerization of *iso*-butylene and styrene, in the presence of a trifunctional initiator such as 1,3,5-*tris*-(2-propyl-2-chloro)benzene. The process, which can be catalyzed by titanium tetrachloride, can be summarized schematically as Reaction IV:

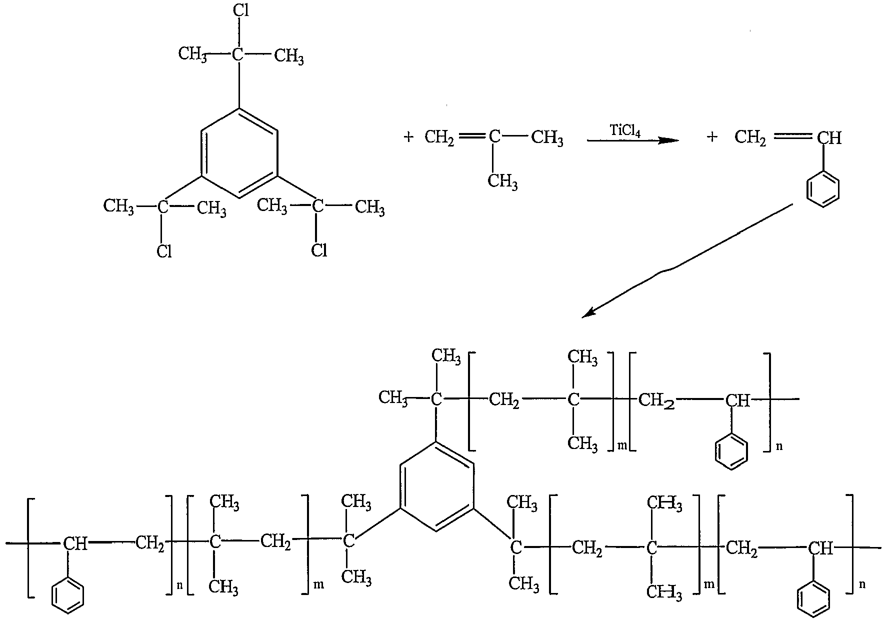

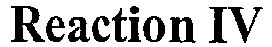

This reaction is done under an inert atmosphere at temperatures of -120 to 0°C in a suitable solvent such as cyclohexane, hexane, heptane, methylene chloride, benzene, or toluene for reaction times of 1 to 24 hours. Further details may be found in Kennedy, J.P. et al. "Design of Polymers by Carbocationic Molecular Engineering," Hanser, NY, Munich (1992).

The viscosity of polymeric solutions containing one of the star polymers described above is expected to be lower than that of polymeric solutions containing the same polymers in a non-star form, with about the same concentration of the polymer in both kinds of solutions. Greater branching will generally compact the packing of the polymer in solution, with the result that, at equal molecular weights, the star polymer will have lower intrinsic viscosity.

The number averaged molecular weight determined chromatographically (e.g., by gel-permeation chromatography method or GPC) (M_{n GPC}) can differ from the star polymers described above from the molecular weight determined osmometrically (M_{n osm}). The ratio M_{n GPC}/ M_{n osm} can be within the range of between about 0.5 and about 1. For the same polymers in the non-star form the ratio can be between about 0.7 and about 1.2.

Any layer of the stent coating can contain any amount of the star polymer(s) described above, or a blend of more than one of such polymers. If less than 100% of the layer is made of the star polymer(s) described above, alternative polymers other than star polymers, can comprise the balance. Examples of the alternative polymers that can be used include polyacrylates, such as poly(butyl methacrylate), poly(ethyl methacrylate), poly(ethyl methacrylate-co-butyl methacrylate), poly(acrylonitrile), poly(ethylene-co-methyl methacrylate), poly(acrylonitrile-co-styrene), and poly(cyanoacrylates); fluorinated polymers and/or copolymers, such as poly(vinylidene fluoride) and poly(vinylidene fluoride-co-hexafluoro propene); poly(N-vinyl pyrrolidone); polyorthoester; polyanhydride; poly(glycolic acid-co-trimethylene carbonate); polyphosphoester; polyphosphoester urethane; poly(amino acids); co-poly(ether-esters); polyalkylene oxalates; polyphosphazenes; biomolecules, such as fibrin, fibrinogen, cellulose, starch, collagen and hyaluronic acid; polyurethanes; silicones; polyesters; polyolefins; polyisobutylene and ethylene-alphaolefin copolymers; vinyl halide polymers and copolymers, such as polyvinyl chloride; polyvinyl ethers, such as polyvinyl methyl ether; polyvinylidene chloride; polyvinyl ketones; polyvinyl aromatics such as polystyrene; polyvinyl esters such as polyvinyl acetate; copolymers of vinyl monomers with each other and olefins, e.g., poly(ethylene-co-vinyl alcohol) (EVAL); ABS resins; and poly(ethylene-co-vinyl acetate); polyamides such as Nylon 66 and polycaprolactam; alkyd resins; polycarbonates; polyoxymethylenes; polyimides; polyethers, epoxy resins; polyurethanes; rayon; rayon-triacetate; cellulose; cellulose acetate; cellulose butyrate; cellulose acetate butyrate; cellophane; cellulose nitrate; cellulose propionate; cellulose ethers; and carboxymethyl cellulose.

Representative examples of some solvents suitable for making the stent coatings include N,N-dimethylacetamide (DMAC), N,N-dimethylformamide (DMF), tethrahydrofurane (THF), cyclohexanone, xylene, toluene, acetone, *i*-propanol, methyl ethyl ketone, propylene glycol monomethyl ether, methyl butyl ketone, ethyl acetate, *n*-butyl acetate, and dioxane. Some solvent mixtures can be used as well. Representative examples of the mixtures include:
DMAC and methanol (e.g., a 50:50 by mass mixture);
water, i-propanol, and DMAC (e.g., a 10:3:87 by mass mixture);
   i-propanol, and DMAC (e.g., 80:20, 50:50, or 20:80 by mass mixtures);
   acetone and cyclohexanone (e.g., 80:20, 50:50, or 20:80 by mass mixtures);
   acetone and xylene (e.g. a 50:50 by mass mixture);
   acetone, FLUX REMOVER AMS, and xylene (e.g., a 10:50:40 by mass mixture); and
   1,1,2-trichloroethane and chloroform (e.g., an 80:20 by mass mixture).

FLUX REMOVER AMS is trade name of a solvent manufactured by Tech Spray, Inc. of Amarillo, Texas comprising about 93.7% of a mixture of 3,3-dichloro-1,1,1,2,2-pentafluoropropane and 1,3-dichloro-1,1,2,2,3-pentafluoropropane, and the balance of methanol, with trace amounts of nitromethane. Those having ordinary skill in the art will select the solvent or a mixture of solvents suitable for a particular polymer being dissolved.

The therapeutic substance which can be used in the reservoir layer can include any substance capable of exerting a therapeutic or prophylactic effect for a patient. The therapeutic substance may include small molecule substances, peptides, proteins, oligonucleotides, and the like. The therapeutic substance could be designed, for example, to inhibit the activity of vascular smooth muscle cells. It can be directed at inhibiting abnormal or inappropriate migration and/or proliferation of smooth muscle cells to inhibit restenosis.

These bioactive agents can be any agent which is a therapeutic, prophylactic, or diagnostic agent. These agents can have anti-proliferative or anti-inflammmatory properties or can have other properties such as antineoplastic, antiplatelet, anti-coagulant, anti-fibrin, anti-thrombonic, antimitotic, antibiotic, antiallergic, antioxidant as well as cystostatic agents. Examples of suitable therapeutic and prophylactic agents include synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules which bind to complementary DNA to inhibit transcription, and ribozymes. Some other examples of other bioactive agents include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy. Examples of anti-proliferative agents include rapamycin and its functional or structural derivatives, 40-*O*-(2-hydroxy)ethyl-rapamyc in (everolimus), and its functional or structural derivatives, paclitaxel and its functional and structural derivatives. Examples of rapamycin derivatives include methyl rapamycin (ABT-578), 40-*O*-(3-hydroxy)propyl-rapamycin, 40-O-[2-(2-hydroxy)ethoxy]ethyl-rapamycin, and 40-*O*-tetrazole-rapamycin. Examples of paclitaxel derivatives include docetaxel. Examples of antineoplastics and/or antimitotics include methotrexate, azathioprine, vincristine, vinblastine, fluorouracil, doxorubicin hydrochloride (e.g. Adriamycin^{®} from Pharmacia & Upjohn, Peapack N.J.), and mitomycin (e.g. Mutamycin^{®} from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as Angiomax a (Biogen, Inc., Cambridge, Mass.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor^{®} from Merck & Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetic, 4-amino-2,2,6,6-tetramethylpiperidine-1-oxyl (4-amino-TEMPO), estradiol, anticancer agents, dietary supplements such as various vitamins, and a combination thereof. Examples of anti-inflammatory agents including steroidal and non-steroidal anti-inflammatory agents include tacrolimus, dexamethasone, clobetasol, combinations thereof. Examples of such cytostatic substance include angiopeptin, angiotensin converting enzyme inhibitors such as captopril (e.g. Capoten^{®} and Capozide^{®} from Bristol-Myers Squibb Co., Stamford, Conn.), cilazapril or lisinopril (e.g. Prinivil^{®} and Prinzide^{®} from Merck & Co., Inc., Whitehouse Station, NJ). An example of an antiallergic agent is permirolast potassium. Other therapeutic substances or agents which may be appropriate include alpha-interferon, bioactive RGD, and genetically engineered epithelial cells. The foregoing substances can also be used in the form of prodrugs or co-drugs thereof. The foregoing substances are listed by way of example and are not meant to be limiting. Other active agents which are currently available or that may be developed in the future are equally applicable.

The dosage or concentration of the bioactive agent required to produce a favorable therapeutic effect should be less than the level at which the bioactive agent produces toxic effects and greater than the level at which non-therapeutic results are obtained. The dosage or concentration of the bioactive agent required to inhibit the desired cellular activity of the vascular region can depend upon factors such as the particular circumstances of the patient; the nature of the tissues being delivered to; the nature of the therapy desired; the time over which the ingredient administered resides at the vascular site; and if other active agents are employed, the nature and type of the substance or combination of substances. Therapeutic effective dosages can be determined empirically, for example by infusing vessels from suitable animal model systems and using immunohistochemical, fluorescent or electron microscopy methods to detect the agent and its effects, or by conducting suitable in vitro studies. Standard pharmacological test procedures to determine dosages are understood by one of ordinary skill in the art.

The coatings and methods of the present invention have been described with reference to a stent, such as a balloon expandable or self-expandable stent. The use of the coating is not limited to stents, however, and the coating can also be used with a variety of other medical devices. Examples of the implantable medical device that can be used in conjunction with the embodiments of this invention include stent-grafts, grafts (e.g., aortic grafts), artificial heart valves, cerebrospinal fluid shunts, pacemaker electrodes, axius coronary shunts and endocardial leads (e.g., FINELINE and ENDOTAK, available from Guidant Corporartion). The underlying structure of the device can be of virtually any design. The device can be made of a metallic material or an alloy such as, but not limited to, cobalt-chromium alloys (e.g., ELGILOY), stainless steel (316L), "MP35N," "MP20N," ELASTINITE (Nitinol), tantalum, tantalum-based alloys, nickel-titanium alloy, platinum, platinum-based alloys such as, e.g., platinum-iridium alloy, iridium, gold, magnesium, titanium, titanium-based alloys, zirconium-based alloys, or combinations thereof. Devices made from bioabsorbable or biostable polymers can also be used with the embodiments of the present invention.

"MP35N" and "MP20N" are trade names for alloys of cobalt, nickel, chromium and molybdenum available from Standard Press Steel Co. of Jenkintown, Pennsylvania. "MP35N" consists of 35% cobalt, 35% nickel, 20% chromium, and 10% molybdenum. "MP20N" consists of 50% cobalt, 20% nickel, 20% chromium, and 10% molybdenum.

### Examples

The following examples are provided to further illustrate embodiments of the present invention.

### Example 1. Making the polymer (A) as shown be Reaction I above.

To a 500 ml flask equipped with magnetic stirrer, oil bath, distillation column with receiver, vacuum line, and argon inlet is added 125 ml of dry toluene, D,L-lactide (125 gm, 0.868 mole), and trimethylolpropane (0.2 gm, 0.0015 mole). Under reduced pressure and with stirring, the toluene is distilled off at 80°C to remove water. After purging with argon, another 125 ml portion of dry toluene is added and the process repeated. A final 125 ml of dry toluene is added with stannous octoate (0.608 gm, 0.0015 mole) and the reaction mixture heated with stirring to 90°C for 14 hours. After cooling to ambient temperature, the reaction mixture is slowly poured into 2 liters of cold methanol with gentle stirring. The polymer is isolated by filtration and dried under vacuum at 40°C.

### Example 2. Making the polymer (B) as shown be Reaction II above.

To a 500 ml flask equipped with magnetic stirrer, oil bath, distillation column with receiver, vacuum line, and argon inlet is added 125 ml of dry toluene, D,L-lactide (125 gm, 0.868 mole), and pentaerythritol (0.2 gm, 0.0015 mole). Under reduced pressure and with stirring, the toluene is distilled off at 80°C to remove water. After purging with argon, another 125 ml portion of dry toluene is added and the process repeated. A final 125 ml of dry toluene is added with stannous octoate (0.608 gm, 0.0015 mole) and the reaction mixture heated with stirring to 90°C for 14 hours. After cooling to ambient temperature, the reaction mixture is slowly poured into 2 liters of cold methanol with gentle stirring. The polymer is isolated by filtration and dried under vacuum at 40°C.

### Example 3. Making the polymer (C) as shown for Reaction III above.

To a 250 ml 3-necked flask equipped with magnetic stirrer, vacuum line, argon inlet, and oil bath is added 125 ml of dry toluene, n-butyl methacrylate (25 gm, 0.176 mole), 1,3,5-*tris*-cyclohexanoyl-2-bromo-isobutyrate (0.112 gm, 3.35 x 10⁻⁴ mole) and 2,2'-bipyridine (0.105 gm, 6.7 x 10⁻⁴ mole,). After dissolution by stirring, the reaction mixture is subjected to three freeze thaw cycles using a liquid nitrogen bath while pulling vacuum. After a final purge with argon, cuprous bromide (0.048 gm, 3.35 x 10⁻⁴ mole) is added and the temperature slowly raised to 90°C. After stirring for 24 hours, the solution is cooled and the polymer precipitated into 1 liter of methanol. After isolation by filtration, the polymer is dried under vacuum at 40°C.

### Example 5. Stent coating containing star polymer. Polymer II

A first composition is prepared by mixing the following components:
about 2 mass % poly(D,L-lactide-co-caprolactone);
dissolved in a mixture of acetone and cyclohexanone (70% and 30% by weight respectively).

The first composition is applied onto the surface of a bare 12 mm VISION stent (available from Guidant Corporation) by spraying and dried to form a stent primer coating. A spray coater is used, having a 0.014 round nozzle maintained at ambient temperature with a feed pressure of about 0.2 atm (about 3 psi) and an atomization pressure of about 1.3 atm (about 20 psi). About 20 µg of the wet coating is applied per pass. Between the passes, the coating was dried at about 50°C for about 10 seconds. Following the last pass, the coating was baked at about 60°C for about 1 hour, yielding a dry primer layer. The dry primer layer contains about 80 µg of poly(D,L-lactide-co-caprolactone).

A second composition was prepared by mixing the following components:
about 6 mass % the polymer of example 2; and
about 2 mass % EVEROLIMUS; and
the balance, a mixture of acetone and cyclohexanone (30% and 70% respectively.

The second composition was applied onto the dry primer layer. Using the same coating technique, about 60 µg of the wet coating is applied per pass. Between the passes, the coating is dried at about 50°C for about 10 seconds. Following the last pass, the coating is baked at about 50°C for about 1 hour, yielding a dry reservoir layer. Due to the high percent solids that can be sprayed, only 8 passes are required. The dry reservoir layer contains about 430 µg of star polymer B and 140 µg of everolimus. The total weight of the coating is about 570 µg.

## Claims

1. An article comprising:
an implantable substrate; and
a coating deposited on at least a portion of the substrate, wherein the coating comprises:
a star polymer;
a comb polymer wherein the sidechains are at least 10% of the contour length of the polymer backbone; or
a brush polymer wherein the sidechains are at least 10% of the contour length of the polymer backbone,
wherein the star polymer, the comb polymer or the brush polymer each comprise any one or any combination of biologically absorbable polymers and durable polymers,
wherein the star polymer comprises any one or any combination of poly(D,L-lactic acid), poly(D-lactic acid), poly(L-lactic acid), poly(L-lactide-co-D,L-lactide), poly(glycolide), poly(D,L-lactide-co-glycolide), poly(caprolactone), poly(D,L-lactide-co-caprolactone), poly(L-lactide-co-caprolactone), poly(glycolide-co-caprolactone), poly(3-hydroxybutyrate), poly(4-hydroxybutyrate), poly(3-hydroxyvalerate), poly(hydroxybutyrate-co-valerate), poly(dioxanone), poly(trimethylene carbonate), poly(D,L-lactide-co-trimethylene carbonate), n-star poly(n-butyl methacrylate) and n-star poly(isobutylene-co-block-styrene), and
wherein the comb polymer or the brush polymer each comprise any one or any combination of poly(D,L-lactic acid), poly(D-lactic acid), poly(L-lactic acid), poly(L-lactide-co-D,L-lactide), poly(glycolide), poly(D,L-lactide-co-glycolide), poly(caprolactone), poly(D,L-lactide-co-caprolactone), poly(L-lactide-co-caprolactone), poly(glycolide-co-caprolactone), poly(3-hydroxybutyrate), poly(4-hydroxybutyrate), poly(3-hydroxyvalerate), poly(hydroxybutyrate-co-valerate), poly(dioxanone), poly(trimethylene carbonate), and poly(D,L-lactide-co-trimethylene carbonate).

2. The article of Claim 1 wherein the implantable substrate is a stent.

3. The article of Claim 1 wherein the star polymer comprises n-star poly(D,L-lactide).

4. The article of Claim 1 wherein the star polymer comprises any one of any combination of 3-armed poly(D,L-lactides), 4-armed poly(D,L-lactides), 5-armed poly(D,L-lactides), or 6-armed poly(D,L-lactides).

5. The article of Claim 1 wherein the star polymer comprises any one or any combination of polymers having the formula of Polymer I or Polymer II: 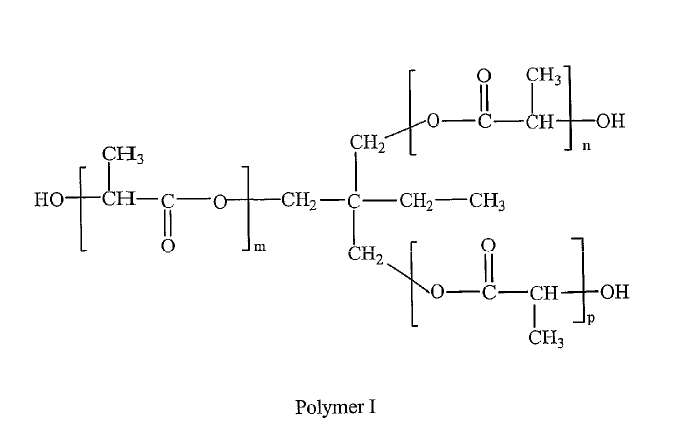 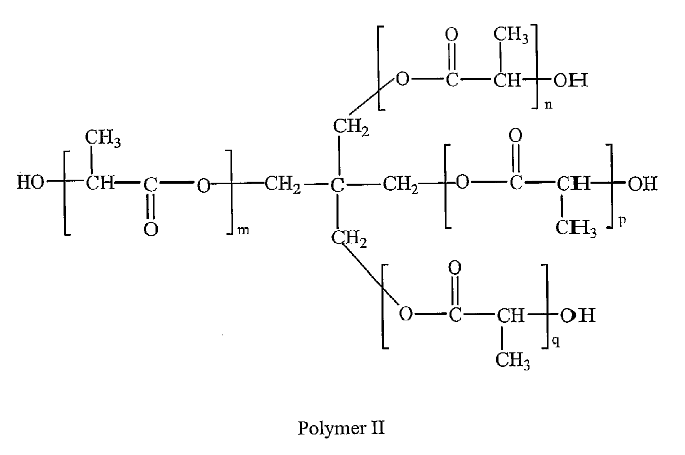 wherein m,n,p and q are integers that can be the same or different.

6. The article of Claim 1 wherein the star polymer is a product of
ring-opening (co)polymerization,
radical (co)polymerization,
ionic (co)polymerization,
living cationic (co)polymerization,
anionic polymerization, or
living polymerization techniques,
wherein the polymerization reacts one or more monomers.

7. The article of Claim 6 wherein living polymerization techniques include atom transfer (co)polymerization, metallocene catalysis, Ziegler-Natta catalysis, nitroxide mediated techniques, or iniferter techniques.

8. The article of Claim 1 wherein the ratio between the number averaged molecular weight of the star polymer measured chromatographically and the number averaged molecular weight of the star polymer measured osmometrically is from 0.5 to 1.0.

9. The article of Claim 1 wherein the comb polymer comprises 1 to 1000 sidechains.

10. The article of Claim 1 wherein the comb polymer comprises 5 to 500 sidechains.

11. The article of Claim 1 wherein the sidechains are at least 40% of the contour length of the polymer backbone.

12. The article of Claim 1 wherein the brush polymer comprises 1 to 1000 sidechains.

13. The article of Claim 1 wherein the brush polymer comprises 5 to 500 sidechains.

14. The article of Claim 1 wherein the star polymer, brush polymer or comb polymer comprises homopolymers, copolymers or terpolymers.

15. The article of Claim 14 wherein the homopolymers, copolymers, or terpolymers are linear.

16. The article of Claim 2, wherein the coating comprises a star polymer.

17. The article of Claim 2, wherein the coating comprises a star polymer comprising any one or any combination of polymers having the formula of Polymer I or Polymer II: 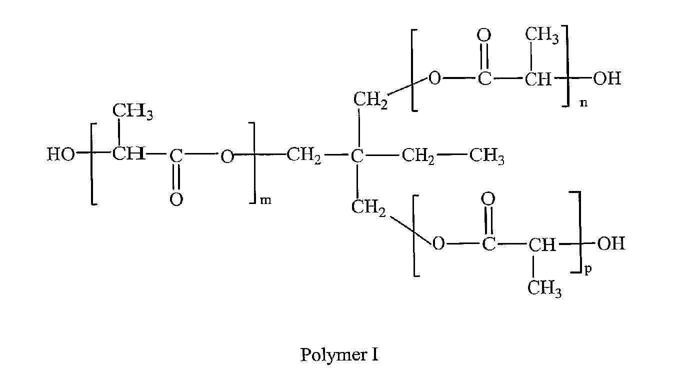 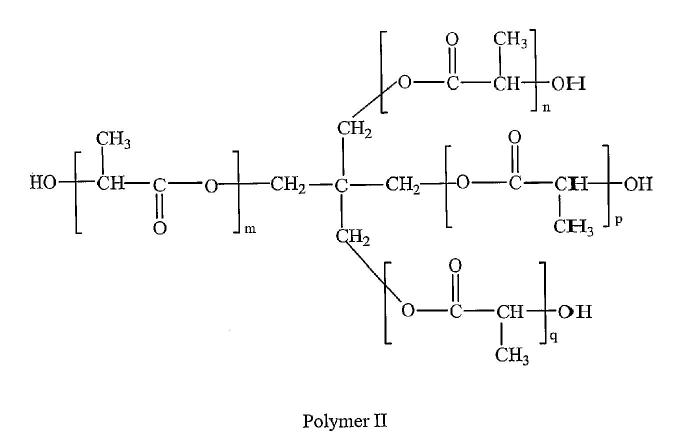 wherein m,n,p and q are integers that can be the same or different.

18. The article of Claim 2, wherein the coating comprises a comb polymer.

19. The article of Claim 2, wherein the coating comprises a brush polymer.

20. A method for fabricating an article according to any of Claims 1 to 19, comprising depositing the coating on the at least a portion of the implantable substrate.

21. The method of Claim 20 additionally including polymerizing at least one monomer using
ring-opening (co)polymerization,
radical (co)polymerization,
ionic (co)polymerization,
living cationic (co)polymerization,
anionic polymerization, or
living polymerization techniques.

22. The method of Claim 21 wherein living polymerization techniques include atom transfer (co)polymerization, metallocene catalysis, Ziegler-Natta catalysis, nitroxide mediated techniques, or iniferter techniques.

## Patentansprüche

1. Erzeugnis, das Folgendes aufweist:
ein implantierbares Substrat; und
eine Beschichtung, die auf wenigstens einem Abschnitt des Substrats abgeschieden ist, wobei die Beschichtung Folgendes aufweist:
ein Sternpolymer;
ein Kammpolymer, wobei die Seitenketten wenigstens 10% der Konturlänge der Hauptkette des Polymers betragen; oder
ein Bürstenpolymer, wobei die Seitenketten wenigstens 10% der Konturlänge der Hauptkette des Polymers betragen,
wobei das Sternpolymer, das Kammpolymer oder das Bürstenpolymer jeweils eines von oder eine Kombination aus biologisch absorbierbaren Polymeren und beständigen Polymeren aufweisen,
wobei das Sternpolymer eines der Folgenden oder eine Kombination aus den Folgendes aufweist: Poly(D, L-Milchsäure), Poly(D-Milchsäure), Poly-(L-Milchsäure), Poly(L-Lactid-co-D, L-Lactid), Poly(Glycolid), Poly(D,L-Lactid-co-Glycolid), Poly(Caprolacton), Poly(D,L-Lactid-co-Caprolacton), Poly(L-Lactid-co-Caprolacton), Poly(Glycolid-co-Caprolacton), Poly(3-Hydroxybutyrat), Poly(4-Hydroxybutyrat), Poly(3-Hydroxyvalerat), Poly(Hydroxy-butyrat-co-Valerat), Poly(Dioxanon), Poly(Trimethylencarbonat), Poly(D,L-Lactid-co-Trimethy-lencarbonat), n-stern-Poly(n-Butylmethacrylat) und n-stern-Poly(Isobutylen-co-block-Styren),
und
wobei das Kammpolymer oder das Bürstenpolymer jeweils eines der Folgenden oder eine Kombination aus den Folgenden aufweisen: Poly(D, L-Milchsäure), Poly(D-Milchsäure), Poly-(L-Milchsäure), Poly(L-Lactid-co-D, L-Lactid), Poly(Glycolid), Poly(D,L-Lactid-co-Glycolid), Poly(Caprolacton), Poly(D,L-Lactid-co-Caprolacton), Poly(L-Lactid-co-Caprolacton), Poly(Glycolid-co-Caprolacton), Poly(3-Hydroxybutyrat), Poly(4-Hydroxybutyrat), Poly(3-Hydroxyvalerat), Poly(Hydroxybutyrat-co-Valerat), Poly(Dioxanon), Poly(Trimethylencarbonat) und Poly(D,L-Lactid-co-Trimethylencarbonat).

2. Erzeugnis nach Anspruch 1, wobei das implantierbare Substrat ein Stent ist.

3. Erzeugnis nach Anspruch 1, wobei das Sternpolymer n-stern-Poly(D,L-Lactid) aufweist.

4. Erzeugnis nach Anspruch 1, wobei das Sternpolymer eines der Folgenden oder eine Kombination aus den Folgenden aufweist: 3-armige Poly(D,L-Lactide), 4-armige Poly(D,L-Lactide), 5-armige Poly(D,L-Lactide) oder 6-armige Poly(D,L-Lactide).

5. Erzeugnis nach Anspruch 1, wobei das Sternpolymer eines der folgenden Polymere oder eine Kombination aus Polymeren mit der Formel von Polymer I oder Polymer II aufweist: 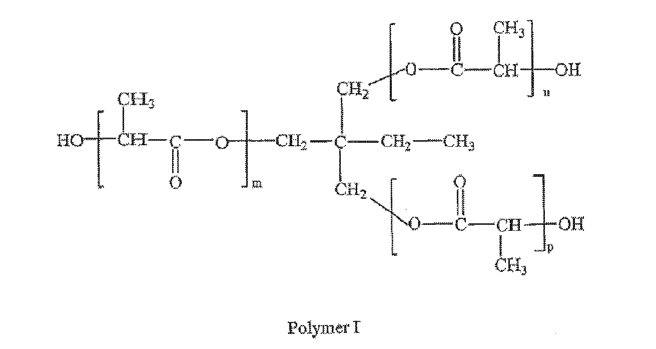 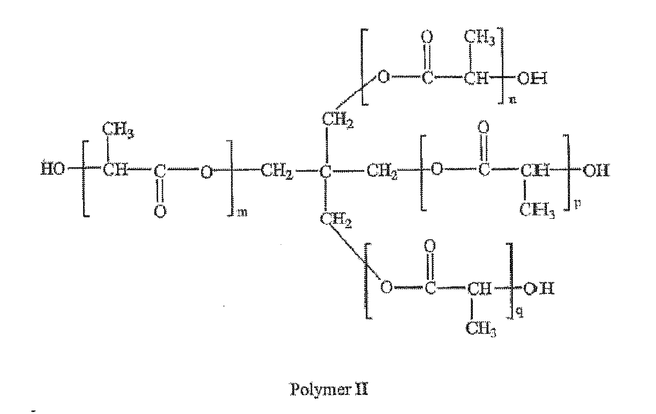 wobei m, n, p und q Ganzzahlen sind, die gleich oder unterschiedlich sein können.

6. Erzeugnis nach Anspruch 1, wobei das Sternpolymer ein Produkt aus
einer ringöffnenden (Co-)Polymerisation,
einer radikalischen (Co-)Polymerisation,
einer ionischen (Co-)Polymerisation,
einer lebenden kationischen (Co-)Polymerisation,
einer anionischen Polymerisation oder
von lebenden Polymerisationstechniken ist,
wobei die Polymerisation ein oder mehrere Monomere zur Reaktion bringt.

7. Erzeugnis nach Anspruch 6, wobei lebende Polymerisationstechniken eine Atomtransfer-(Co-)Polymerisation, eine Metallocen-Katalyse, eine Ziegler-Natta-Katalyse, Nitroxidvermittelte Techniken oder Iniferter-Techniken aufweisen.

8. Erzeugnis nach Anspruch 1, wobei das Verhältnis zwischen dem zahlengemittelten Molekulargewicht des Sternpolymers, das chromatographisch gemessen wird, und dem zahlengemittelten Molekulargewicht des Sternpolymers, das osmometrisch gemessen wird, 0,5 bis 1,0 beträgt.

9. Erzeugnis nach Anspruch 1, wobei das Kammpolymer 1 bis 1000 Seitenketten aufweist.

10. Erzeugnis nach Anspruch 1, wobei das Kammpolymer 5 bis 500 Seitenketten aufweist.

11. Erzeugnis nach Anspruch 1, wobei die Seitenketten wenigstens 40% der Konturlänge der Hauptkette des Polymers betragen.

12. Erzeugnis nach Anspruch 1, wobei das Bürstenpolymer 1 bis 1000 Seitenketten aufweist.

13. Erzeugnis nach Anspruch 1, wobei das Bürstenpolymer 5 bis 500 Seitenketten aufweist.

14. Erzeugnis nach Anspruch 1, wobei das Sternpolymer, Bürstenpolymer oder Kammpolymer Homopolymere, Copolymere oder Terpolymere aufweist.

15. Erzeugnis nach Anspruch 14, wobei die Homopolymere, Copolymere oder Terpolymere linear sind.

16. Erzeugnis nach Anspruch 2, wobei die Beschichtung ein Sternpolymer aufweist.

17. Erzeugnis nach Anspruch 2, wobei die Beschichtung ein Sternpolymer aufweist, das eines der folgenden Polymere oder eine Kombination aus Polymeren mit der Formel von Polymer I oder Polymer II aufweist: 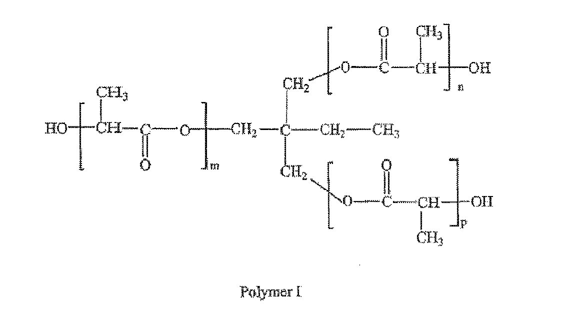 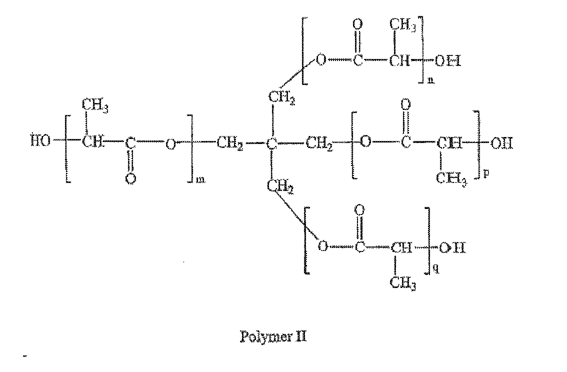 wobei m, n, p und q Ganzzahlen sind, die gleich oder unterschiedlich sein können.

18. Erzeugnis nach Anspruch 2, wobei die Beschichtung ein Kammpolymer aufweist.

19. Erzeugnis nach Anspruch 2, wobei die Beschichtung ein Bürstenpolymer aufweist.

20. Verfahren zur Herstellung eines Erzeugnisses nach einem der Ansprüche 1 bis 19, wobei das Verfahren ein Abscheiden der Beschichtung auf dem wenigstens einen Abschnitt des implantierbaren Substrats aufweist.

21. Verfahren nach Anspruch 20, das zusätzlich ein Polymerisieren von wenigstens einem Monomer unter Verwendung
einer ringöffnenden (Co-)Polymerisation,
einer radikalischen (Co-)Polymerisation,
einer ionischen (Co-)Polymerisation,
einer lebenden kationischen (Co-)Polymerisation,
einer anionischen Polymerisation oder
von lebenden Polymerisationstechniken
aufweist.

22. Verfahren nach Anspruch 21, wobei lebende Polymerisationstechniken eine Atomtransfer-(Co-)Polymerisation, eine Metallocen-Katalyse, eine Ziegler-Natta-Katalyse, Nitroxidvermittelte Techniken oder Iniferter-Techniken aufweisen.

## Revendications

1. Article comprenant :
un substrat implantable ; et
un revêtement déposé sur au moins une partie du substrat, le revêtement comprenant :
un polymère en étoile ;
un polymère en peigne dans lequel les chaînes latérales représentent au moins 10 % de la longueur de contour du squelette du polymère ; ou
un polymère en brosse dans lequel les chaînes latérales représentent au moins 10 % de la longueur de contour du squelette polymère,
le polymère en étoile, le polymère en peigne ou le polymère en brosse comprenant chacun un quelconque ou une quelconque combinaison des polymères biologiquement absorbables et polymères durables,
dans lequel le polymère en étoile comprend un quelconque ou une quelconque combinaison des poly(acide D,L-lactique), poly(acide D-lactique), poly(acide L-lactique), poly(L-lactide-co-D,L-lactide), poly(glycolide), poly(D,L-lactide-co-glycolide), poly(caprolactone), poly-(D, L-lactide-co-caprolactone), poly (L-lactide-co-caprolactone), poly(glycolide-co-caprolactone), poly(3-hydroxybutyrate), poly(4-hydroxybutyrate), poly(3-hydroxyvalérate), poly-(hydroxybutyrate-co-valérate), poly(dioxanone), poly-(carbonate de triméthylène), poly(carbonate de D,L-lactide-co-triméthylène), poly(méthacrylate de n-butyle) n-étoilé et poly(isobutylène-co-bloc-styrène) n-étoilé, et
dans lequel le polymère en peigne ou le polymère en brosse comprend un quelconque ou une quelconque combinaison de poly(acide D,L-lactique), poly(acide D-lactique), poly(acide L-lactique), poly(L-lactide-co-D,L-lactide), poly(glycolide), poly(D,L-lactide-co-glycolide), poly-(caprolactone), poly(D,L-lactide-co-caprolactone), poly(L-lactide-co-caprolactone), poly(glycolide-co-caprolactone), poly(3-hydroxybutyrate), poly(4-hydroxybutyrate), poly(3-hydroxyvalérate), poly(hydroxybutyrate-co-valérate), poly-(dioxanone), poly(carbonate de triméthylène) et poly-(carbonate de D,L-lactide-co-triméthylène).

2. Article selon la revendication 1, dans lequel le substrat implantable est une endoprothèse.

3. Article selon la revendication 1, dans lequel le polymère en étoile comprend un poly(D,L-lactide) n-étoilé.

4. Article selon la revendication 1, dans lequel le polymère en étoile comprend un quelconque ou une quelconque combinaison de poly(D,L-lactides) à 3 bras, poly(D,L-lactides) à 4 bras, poly(D,L-lactides) à 5 bras ou poly-(D,L-lactides) à 6 bras.

5. Article selon la revendication 1, dans lequel le polymère en étoile comprend un quelconque ou une quelconque combinaison des polymères ayant la formule du polymère I ou du polymère II : 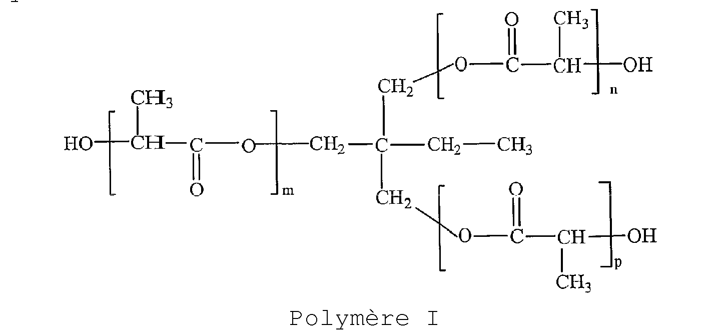 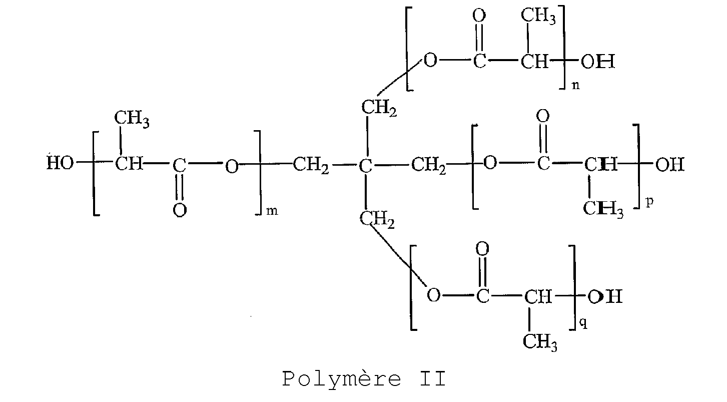 où m, n, p et q sont des nombres entiers qui peuvent être identiques ou différents.

6. Article selon la revendication 1, dans lequel le polymère en étoile est un produit de :
(co)polymérisation par ouverture de cycle,
(co)polymérisation radicalaire,
(co)polymérisation ionique,
(co)polymérisation cationique vivante,
polymérisation anionique, ou
techniques de polymérisation vivante,
la polymérisation faisant réagir un ou plusieurs monomères.

7. Article selon la revendication 6, dans lequel les techniques de polymérisation vivante comprennent la (co)polymérisation par transfert d'atomes, la catalyse métallocène, la catalyse de Ziegler-Natta, les techniques à médiation par nitroxyde ou les techniques à base d'iniferter.

8. Article selon la revendication 1, dans lequel le rapport entre le poids moléculaire moyen en nombre du polymère en étoile mesuré par chromatographie et le poids moléculaire moyen en nombre du polymère en étoile mesuré par osmométrie est de 0,5 à 1,0.

9. Article selon la revendication 1, dans lequel le polymère en peigne comprend 1 à 1000 chaînes latérales.

10. Article selon la revendication 1, dans lequel le polymère en peigne comprend 5 à 500 chaînes latérales.

11. Article selon la revendication 1, dans lequel les chaînes latérales représentent au moins 40 % de la longueur de contour du squelette du polymère.

12. Article selon la revendication 1, dans lequel le polymère en brosse comprend 1 à 1000 chaînes latérales.

13. Article selon la revendication 1, dans lequel le polymère en brosse comprend 5 à 500 chaînes latérales.

14. Article selon la revendication 1, dans lequel le polymère en étoile, le polymère en brosse ou le polymère en peigne comprend des homopolymères, des copolymères ou des terpolymères.

15. Article selon la revendication 14, dans lequel les homopolymères, les copolymères ou les terpolymères sont linéaires.

16. Article selon la revendication 2, dans lequel le revêtement comprend un polymère en étoile.

17. Article selon la revendication 2, dans lequel le revêtement comprend un polymère en étoile comprenant un quelconque ou une quelconque combinaison des polymères ayant la formule du polymère I ou du polymère II : 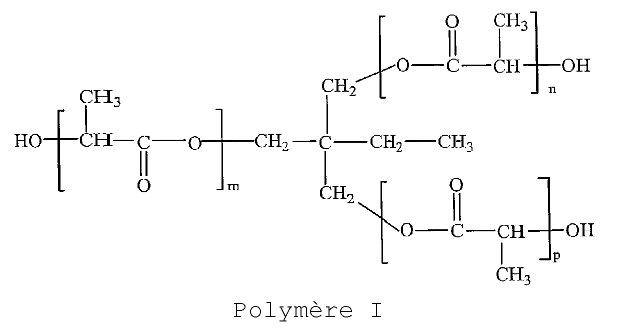 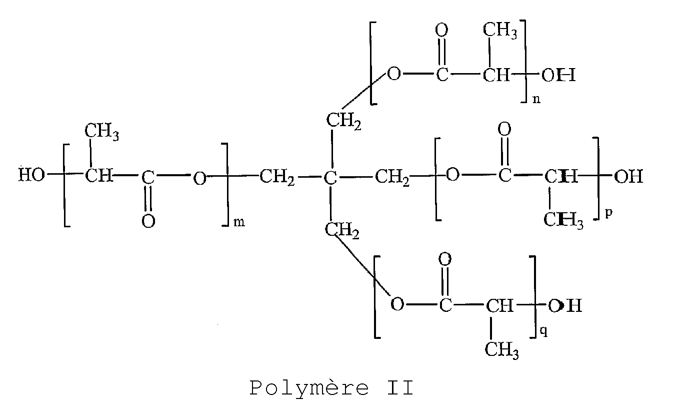 où m, n, p et q sont des nombres entiers qui peuvent être identiques ou différents.

18. Article selon la revendication 2, dans lequel le revêtement comprend un polymère en peigne.

19. Article selon la revendication 2, dans lequel le revêtement comprend un polymère en brosse.

20. Procédé de fabrication d'un article selon l'une quelconque des revendications 1 à 19, comprenant le dépôt du revêtement sur au moins une partie du substrat implantable.

21. Procédé selon la revendication 20, comprenant en outre la polymérisation d'au moins un monomère en utilisant :
une (co)polymérisation par ouverture de cycle,
une (co)polymérisation radicalaire,
une (co)polymérisation ionique,
une (co)polymérisation cationique vivante,
une polymérisation anionique, ou
des techniques de polymérisation vivante.

22. Procédé selon la revendication 21, dans laquelle les techniques de polymérisation vivante comprennent la (co)polymérisation par transfert d'atomes, la catalyse métallocène, la catalyse de Ziegler-Natta, les techniques à médiation par nitroxyde ou les techniques à base d'iniferter.
